**Europäisches Patentamt**

(19) **European Patent Office**

**Office européen des brevets**

(11) Publication number : **0 506 866 B1**

(12) # EUROPEAN PATENT SPECIFICATION

(45) Date of publication of patent specification :
**20.10.93 Bulletin 93/42**

(51) Int. Cl.⁵ : **C12N 9/98**

(21) Application number : **91902671.6**

(22) Date of filing : **21.12.90**

(86) International application number :
**PCT/DK90/00341**

(87) International publication number :
**WO 91/09943 11.07.91 Gazette 91/15**

(54) **METHOD FOR CRYSTALLIZATION OF ENZYMES.**

(30) Priority : **21.12.89 DK 6540/89**
**05.04.90 DK 847/90**
**05.04.90 DK 848/90**

(43) Date of publication of application :
**07.10.92 Bulletin 92/41**

(45) Publication of the grant of the patent :
**20.10.93 Bulletin 93/42**

(84) Designated Contracting States :
**AT BE CH DE DK ES FR GB GR IT LI LU NL SE**

(56) References cited :
**WO-A-89/08703**
**DE-A- 1 959 603**
**US-A- 4 699 882**

(73) Proprietor : **NOVO NORDISK A/S**
**Novo Allé**
**DK-2880 Bagsvaerd (DK)**

(72) Inventor : **NIELSEN, Niels-Viktor**
**Ryegade 5**
**DK-4060 Kirke Saaby (DK)**
Inventor : **NIELSEN, Torben Kjaersgaard**
**Tinggaardsvaenget 90**
**Tune DK-4000 Roskilde (DK)**

(74) Representative : **Bach, Niels et al**
**c/o Novo Nordisk A/S Patent Dept. Novo Allé**
**DK-2880 Bagsvaerd (DK)**

EP 0 506 866 B1

Jouve, 18, rue Saint-Denis, 75001 PARIS

## Description

The invention encompasses a method for crystallization of enzymes.

Enzymes are usually provided as liquids or solid materials for industrial purposes. When not provided as liquids, they are usually provided as amorphous materials, because the known methods for crystallization of enzymes usually are regarded as too expensive to be used in an industrial scale.

Thus, the purpose of the invention is the provision of a method for crystallization of enzymes, which is simple and cheap, and which is compatible to industrial requirements.

The method according to the invention is characterized by the fact that an aqueous enzyme containing liquid with an enzyme purity above 20% (i.e. the pure enzyme amounts to more than 20% of the total dry matter in the enzyme containing liquid) and with a concentration of pure enzyme protein of at least 5 g/l of enzyme containing liquid is used as a starting material, and that a crystallization agent, which is Na, K, Ca, or Mg formate, acetate or nitrate is added to the starting. material to a final concentration which is smaller than the concentration needed to precipitate the enzymes in an amorphous form.

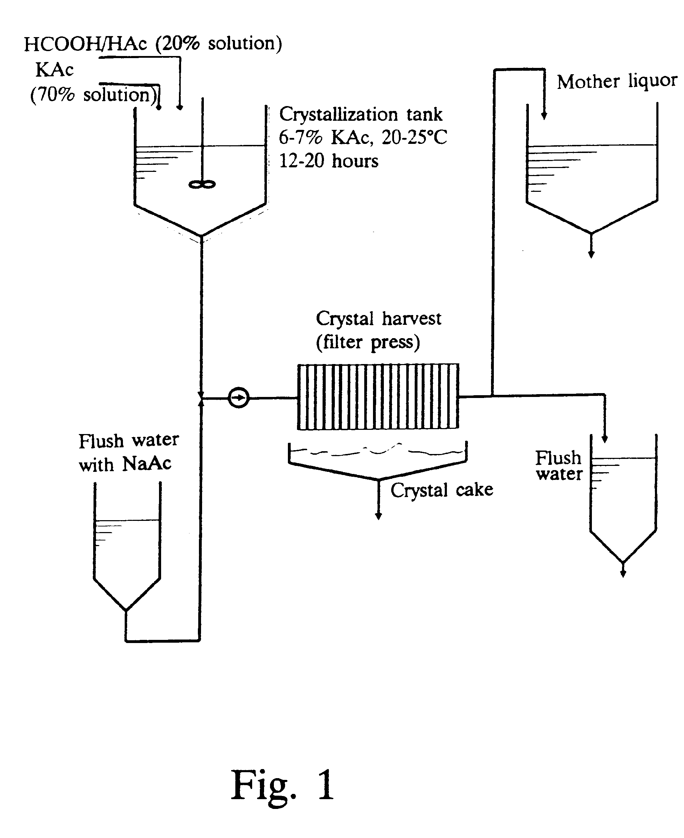

When carried out on an industrial scale, the crystals are separated in a filter, and the crystals are subsequently flushed for purification purposes. Reference is made to Fig. 1.

All crystallization agents used in the method according to the invention are easily soluble salts, i.e. salts, which in pure water at 25°C exhibit a solubility above 5 g/l.

The above indicated starting materials are known in the art, and they can be provided for instance as described in chapter 9 ("Production of Microbial Enzymes") in Microbial Technology, 2nd edition, Vol. 1, Academic Press, Inc., 1979; and thus, an outline of recovery and purification methods for technical grade enzymes can be found in this chapter. Furthermore US patent No. 3,795,583 describes purification methods for enzyme containing culture broths.

Surprisingly it has been found that the method according to the invention which is simple and cheap, can be carried out with a yield of up to 95%, and that it can easily be adopted to industrial practice.

Thus, the starting material for the method according to the invention for crystallization of enzymes is an aqueous enzyme containing liquid with an enzyme purity of above 20% (i.e. the pure enzyme amounts to more than 20% of the total dry matter in the enzyme containing liquid), and with a concentration of enzyme protein of at least 5 g/l of enzyme containing liquid. The time necessary for crystallization is usually between 5 and 12 hours. By addition of crystal seeds in an amount of e.g. 1% the crystallization velocity can be accelerated. The crystals are preferably separated from the supernatant by filtration.

In a preferred embodiment of the method according to the invention the crystallization agent is added to the starting material to a final concentration corresponding to an added amount of crystallization agent of from 0.02 to 1.7M of crystallization agent, preferably from 0.05 to 1.6M, more preferably from 0.10 to 1.5M.

In a preferred embodiment of the method according to the invention the enzyme is a protease, lipase, amylase, cellulase, hemicellulase, pectinase, amidase or oxidase. These enzymes are commonly used as additives in detergents, and thus, when crystalline enzymes are desirable in the detergent industry, this embodiment is preferred. Also protein engineered variants of these enzymes are within the scope of the invention.

In a preferred embodiment of the method according to the invention the enzyme is a protease and the protease is a Subtilisin type protease, preferably Savinase® or a protein engineered variant of Savinase® or Alcalase®. Other examples of such proteases are Alcalase®, Subtilisin NOVO or protein engineered variants of these, which are very commonly used as additives in detergents, and thus, when crystalline Savinase®, Esperase®, Alcalase®, Subtilisin NOVO or protein engineered variants of these is desirable in the detergent industry, this embodiment is preferred.

DK patent application No. 872/86 describes a method for crystallization of an enzyme containing solution, wherein a supersaturated solution is crystallized at a pH close to the isoelectric point of the enzyme.

US 4,699,882 describes a crystallization method specifically directed to glucose isomerase. The crystallization agents used are magnesium sulphate and ammonium sulphate.

WO 89/08703 describes a Subtilisin crystallization process, which as an imperative step uses a crystallization agent, which is a halide salt, and which can only be performed satisfactorily below around 10°C.

Thus, all these prior art methods differ substantially from the method according to the invention.

## EXAMPLES 1 - 4

The starting material for the crystallization of Savinase® crystals is prepared in the following manner.

To 1 kg of Savinase® fermentation broth (US patent No. 3,723,250) are added 7.5 g of $CaCl_2 \cdot 2H_2O$ and 1 litre of water. The pH is adjusted to 8.0 with an aqueous sodium hydroxide solution. The suspension is then flocculated by addition of Superfloc C 521 flocculation agent (approx. 15 g/litre of broth) and Superfloc A 130

2

flocculation agent (approx. 0.2 g/litre of broth). The flocculated suspension is centrifuged and the supernatant is filtered on an appropriate filter sheet in order to obtain a clear liquid. The filtrate is concentrated by evaporation to a value of RI (refractive index) of 12% and then heated to approx. 38°C. At this temperature sodium sulphate (250 g/kg enzyme solution) is added stepwise to the solution with rapid mixing.

The precipitate is filtered off and is reslurried in cold (< 5°C) water (3-5 litres of water/kg of filter cake). The undissolved material is removed by filtration and the clear filtrate is ultra- and diafiltered to a point where the value of the RI (Refractometer Index) dry matter in the permeate is less than 0.5% and the value of the RI dry matter in the concentrate is between 10-16%. The proteolytic activity is 18-22 KNPU/g.

The resulting concentrate exhibits a content of pure Savinase® protease on a dry matter basis above 25% w/w.

To the starting material, i.e. the above indicated concentrate, is added 7% of calcium formate (0.54 M added) serving as a precipitation agent, at 20-25°C and a pH value of approximately 5.0. The mixture is seeded with Savinase® crystals (approx. 1% w/w) and then gently agitated, and after 3-6 hours a powerful precipitate of crystalline Savinase® can be observed. The amount of the crystal sludge is approx. 20% of the total volume and exhibits an enzyme activity of approx. 86 KNPU/g. The precipitation yield on the basis of KNPU is around 81%.

The following Examples 2 - 4 were carried out with the same Savinase® concentrate, seeding procedure, temperature (20-24°C) and pH (5.0). All the generated crystal sludges had an activity of about 80 KNPU/g.

The variable parameters in Examples 2 - 4 appear from the following table.

## Table 1

| Example no. | Precipitation agent | Concentration of added precipitation agent | % sludge | % Yield |
|---|---|---|---|---|
| 2 | Ca(CHOO)$_2$ | 0.38 M | 17.5 | 73 |
| 3 | K(CH$_3$COO) | 0.82 M | 15.0 | 74 |
| 4 | K(CH$_3$COO) | 1.00 M | 16.0 | 78 |

**EXAMPLES 5 - 69**

It has been found that the crystallization process is dependent on the following parameters:

**pH:** with increasing pH the crystal size decreases, and the crystallization velocity increases.

**Salt concentration:** with increasing salt concentration the crystallization yield increases, the crystallization velocity increases, and the crystal size decreases.

**Salt type:** the crystallization performance varies from salt to salt.

**Temperature:** with increasing temperature the crystallization velocity increases, and the crystal size decreases.

**Enzyme concentration:** with increasing enzyme concentration the crystallization velocity increases, the crystallization yield increases, and the crystal size decreases.

**Enzyme purity:** with increasing enzyme purity the crystallization velocity increases, and the crystallization yield increases.

The following examples 5 - 18, which illustrate the above indicated pH dependency and the salt concentration dependency, were carried out with a Savinase® concentrate, made by the method mentioned in Examples 1 - 4. The temperature was kept at 20-25°C and the concentration of enzyme was approx. 55 g/l. The crystallization agent was potassium acetate. The enzyme purity was approximately 35%.

| Example no. | pH | Mol potassium acetate added/l | crystallization start, comment |
|---|---|---|---|
| 5 | 4.0 | 0.8 | no crystallization |
| 6 | 4.5 | 0.8 | 1-2 h, 20-50 $\mu$m crystals |
| 7 | 5.5 | 0.8 | ½-1 h, 10-50 $\mu$m crystals |
| 8 | 6.5 | 0.8 | $\approx$ ½ h, 5-50 $\mu$m crystals |
| 9 | 7.5 | 0.8 | $\approx$ ½ h, 5-10 $\mu$m crystals |

| Example no. | pH | Mol potassium acetate added/l | crystallization yield, comment |
|---|---|---|---|
| 10 | 5.5 | 0.05 | no crystallization |
| 11 | 5.5 | 0.10 | no crystallization |
| 12 | 5.5 | 0.20 | no crystallization |
| 13 | 5.5 | 0.40 | $\approx$ 75%, 10-30 $\mu$m crystals |
| 14 | 5.5 | 0.60 | $\approx$ 84%, 5-20 $\mu$m crystals |
| 15 | 5.5 | 0.80 | $\approx$ 84%, 1-20 $\mu$m crystals |
| 16 | 5.5 | 1.20 | $\approx$ 84%, 1-20 $\mu$m crystals |
| 17 | 5.5 | 1.40 | $\approx$ 87%, 1-10 $\mu$m crystals |
| 18 | 5.5 | >1.60 | amorphous fragments |

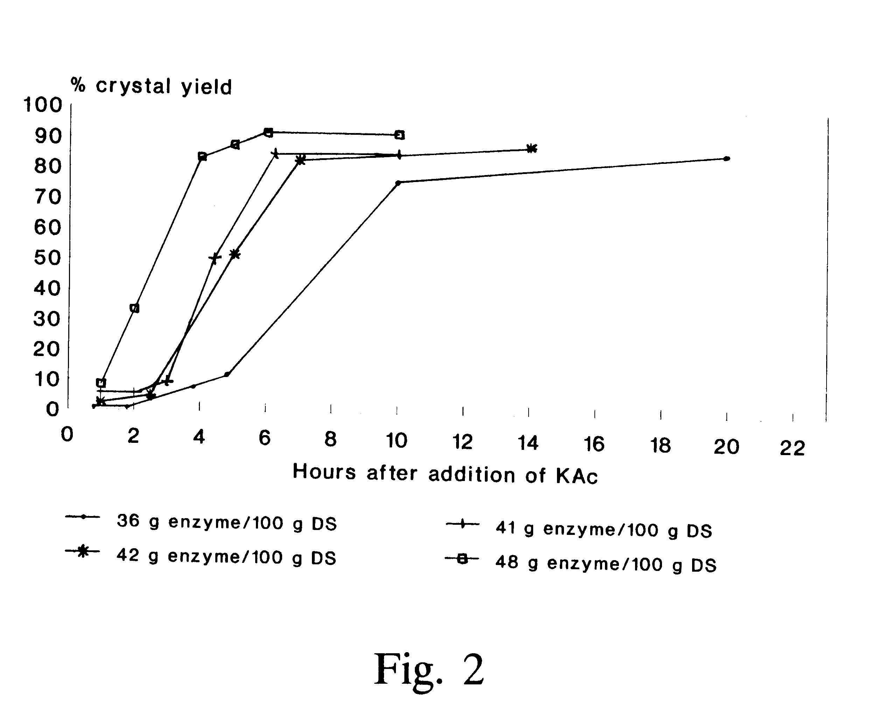

Reference is made to Fig. 2, which shows crystallization performance (Savinase®) as a function of enzyme purity. Fig. 2 shows that the crystallization velocity and the crystallization yield increases with increasing enzyme purity.

The following examples 19-25 which show the crystallization performance as a function of enzyme concentration, were carried out at 20-25°C, with 0.8 mol added potassium acetate/l and at pH 4.9. The enzyme purity is about 38% (DS basis). The Savinase® samples are taken from different stages in the final ultrafiltration in the recovery process mentioned in Examples 1 - 4, corresponding to the values in the column "g enzyme/l".

| Example no. | g enzyme/l | Start of crystallization | Yield |
|---|---|---|---|
| 19 | 8.6 | ÷ | 0% |
| 20 | 17.0 | 2-3 h | 70% |
| 21 | 25.0 | 1½-2 h | 85% |
| 22 | 33.0 | 1½ h | 88% |
| 23 | 41.0 | ½-1 h | 90% |
| 24 | 50.0 | ½-1 h | 92% |
| 25 | 58.0 | ½-1 h | 94% |

The following examples 26-29 which show the crystallization performance as a function of enzyme temperature, are carried out with 0.51 mol added potassium acetate/l, pH 5.5 and an enzyme concentration at about 55 g/l. The enzyme purity is about 36% (DS basis). Maximum crystallization time: 12 hours.

| Example no. | Temperature | Start of crystallization | Yield |
|---|---|---|---|
| 26 | 10°C | > 10 hours | < 1% |
| 27 | 15°C | 3 hours | 78% |
| 28 | 20°C | 2 hours | 85% |
| 29 | 30°C | ¾ hours | 93% |

The following examples 30 - 69 show that the crystallization process can easily be used with other subtilisins such as Alcalase® and Durazym®.

The starting material for the crystallization is prepared by a recovery process which is very similar to that indicated in Examples 1 - 4.

In the case of Alcalase®, during the flocculation step besides $CaCl_2$ about 4 g of $NaAlO_2$ per l of fermentation broth is added. The product will end up with a proteolytic activity at about 3.5 AU/g.

Durazym®, which is a protein engineered version of Savinase®, is prepared analogously to the crystallization of Savinase® in Examples 1 - 4. The product will end up with a proteolytic activity at about 19 DPU/g.

Before crystallization both enzymes have an enzyme purity above 25% measured on dry matter basis.

In the following examples 30-34 the enzyme is Alcalase®, and the pH during the crystallization is 4.5-8.5, the temperature is between 20 and 25°C, the crystallization agent is sodium formate, which is added in a concentration of 1.5 M/l, the enzyme concentration is approximately 66 g/l, and the enzyme purity is 50%.

| Example no. | pH | Start of crystallization | Yield |
|---|---|---|---|
| 30 | 4.5 | 1 hour | 92% |
| 31 | 5.5 | 1 hour | 93% |
| 32 | 6.5 | ½ hour | 95% |
| 33 | 7.5 | ½ hour | ≈ 93% |
| 34 | 8.5 | « ½ hour | ≈ 93% |

In the following examples in which Alcalase® and different crystallization agents are used the pH during the crystallization is between 4.5 and 5.0, the temperature is between 20 and 25°C, the crystallization time is between 6 and 24 hours, the enzyme concentration is approx. 66 g/l and the enzyme purity is 50%.

**Enzyme: Alcalase®**

| Example no. | Salt | M salt added/l | Yield |
|---|---|---|---|
| 35 | $Na(CH_3COO)$ | 0.7 | 35% |
| 36 | $Na(CH_3COO)$ | 1.2 | 70% |
| 37 | $Na(CH_3COO)$ | 1.7 | 78% |
| 38 | $Na(CHOO)$ | 0.9 | 80% |
| 39 | $Na(CHOO)$ | 1.1 | 90% |
| 40 | $Na(CHOO)$ | 1.4 | 96% |
| 41 | $Ca(CHOO)_2$ | 0.4 | 45% |
| 42 | $Ca(CHOO)_2$ | 0.8 | 60% |
| 43 | $Ca(CHOO)_2$ | 0.9 | 78% |

In the following examples which are performed with Alcalase® and with crystallization with sodium formate at different enzyme concentrations the pH during the crystallization is between 4.5 and 5.0, the temperature is between 20 and 25°C, the crystallization time is 12 hours (at 20 to 25°C) plus 12 hours at 10°C. The enzyme concentration is approx. 66 g/l, the enzyme purity is 50%, and the salt is added in a dosage of 1.5 Mol/l.

| Example no. | g enzyme/l | Start of crystallization | Yield |
|---|---|---|---|
| 44 | 19 | 2 hours | 60% |
| 45 | 29 | 1 hour | 81% |
| 46 | 40 | 1 hour | 86% |
| 47 | 50 | ¾ hour | 90% |
| 48 | 58 | < ¾ hour | 93% |

The following examples with Alcalase® which illustrate crystallization performance as a function of temperatures are carried out at pH 4.5 and a temperature between 5 and 30°C. The sodium formate is added in an amount of 1.5 Mol/l. The enzyme concentration is approx. 66 g/l and the enzyme purity is 50%. The crystallization time is 12 hours at the temperature specified plus 12 hours at 10°C.

| Example no. | Temperature | Start of crystallization | Yield |
|---|---|---|---|
| 49 | 5°C | > 12 hours | - |
| 50 | 15°C | > 12 hours | - |
| 51 | 20°C | 1 hour | 90% |
| 52 | 25°C | < ½ hour | ≈ 88% |
| 53 | 30°C | < ½ hour | ≈ 86% |

The following examples which illustrate crystallization performance of Alcalase® as a function of enzyme temperature are carried out at pH 5.0 and at a temeprature between 15 and 30°C. The dosage of calcium formate is 0.8 Mol added /l. The enzyme concentration is approx. 63 g/l and the purity is 50%.
The crystallization time is 12 hours (at the specified temperature) plus 12 hours at 10°C.

| Example no. | Temperature | Start of crystallization | Yield/crystal size |
|---|---|---|---|
| 54 | 15°C | 10 hours | 4% / 50-80 $\mu$m |
| 55 | 20°C | 4 hours | 16% / 40-80 $\mu$m |
| 56 | 25°C | 2¼ hour | 46% / 10-60 $\mu$m |
| 57 | 30°C | < ½ hour | 49% / 15-40 $\mu$m |

The following examples which illustrate crystallization of Alcalase® at different pH values the pH is varied from 4.5 to 8.5, the temperature is between 20 and 25°C, the salt dosage is 0.8 Mol added/l the enzyme concentration is approx. 66 g/l and the enzyme purity is 50%. Salt: pottassium acetate.

| Example no. | pH | Start of crystallization | Yield/crystal size |
|---|---|---|---|
| 58 | 4.5 | 3½ hour | 55% / 30-70 $\mu$m |
| 59 | 5.5 | 1½ hour | $\approx$ 60%[*] / 1-10 $\mu$m |
| 60 | 6.5 | 1½ hour | $\approx$ 60%[*] / 1-10 $\mu$m |
| 61 | 7.5 | 1 hour | $\approx$ 60%[*] / 1-10 $\mu$m |
| 62 | 8.5 | ¼ hour | $\approx$ 60%[*] / 1-10 $\mu$m |

[*] The crystals are very small and therefore hard to separate from the mother liquor

The following examples with Durazym®, which show crystallization with different crystallization agents are carried out at pH 4.9 and a temperature between 20 and 25°C. The enzyme concentration is approx. 48 g/l and the enzyme purity is approx. 30%.

**Enzyme: Durazym®**

| Example no. | Salt | M salt added/l | Yield |
|---|---|---|---|
| 63 | Na(CHOO) | 0.6 | 42% |
| 64 | Na(CHOO) | 1.1 | 90% |
| 65 | Na(CHOO) | 1.5 | 96% |
| 66 | K(CH$_3$COO) | 0.6 | 62% |
| 67 | K(CH$_3$COO) | 0.8 | 83% |
| 68 | K(CH$_3$COO) | 1.2 | 86% |

**EXAMPLE 69**

Crystallization of *Candida* lipase B

*Candida* lipase B is described in WO 88/02775, from which is appears that this lipase can be produced by means of *Candida antarctica* DMS 3855.

After fermentation the culture broth is pretreated by addition of 0.25 l of water per liter of culture broth. pH is adjusted to 8.0 by means of an aqueous solution of NaOH. The suspension is filtered on a drum filter, which is precoated with Dicalite 4208 kiselguhr. The filtrate is subsequently filtered on appropriate filter plates in order to obtain a completely clear liquid. Finally an ultrafiltration and a diafiltration is carried out until a value of RI (Refrectometer Index) dry substance in the concentrate of around 12% and until the value of RI dry substance in the permeate is less than 2%. Finally a sterile filtration is carried out. This filtrate which exhibits a lipase activity of approx. 25 KLU/g and a pH of 7.0 is the starting material for the crystallization. To the starting material is added 4-6% salt, which is CH$_3$COOK(KAc) or HCOONa. After a few minutes a powerful precipitate of crys-

EP 0 506 866 B1

talline *Candida* lipase B is formed, and 2-5 hours later the crystalline precipitate amounts to around 20% of the total volume. The crystals exhibit an activity of 65-70 KLU/g, and the yield exceeds 80%. The crystals are small and needle shaped.

## Claims

1. Method for crystallization of enzymes, characterized by the fact that an aqueous enzyme containing liquid with an enzyme purity above 20% (i.e. the pure enzyme amounts to more than 20% of the total dry matter in the enzyme containing liquid) and with a concentration of pure enzyme protein of at least 5 g/l of enzyme containing liquid is used as a starting material, and that a crystallization agent, which is Na, K, Ca, or Mg formate, acetate or nitrate is added to the starting material to a final concentration which is smaller than the concentration needed to precipitate the enzymes in an amorphous form.

2. Method according to Claim 1, wherein the crystallization agent is added to the starting material to a final concentration corresponding to an added amount of crystallization agent of from 0.02 to 1.7M of crystallization agent, preferably from 0.05 to 1.6M, more preferably from 0.10 to 1.5M.

3. Method according to Claim 1 or 2, characterized by the fact, that the enzyme is a protease, lipase, amylase, cellulase, hemicellulase, pectinase, amidase or oxidase.

4. Method according to Claim 3, characterized by the fact that the enzyme is a protease and that the protease is a Subtilisin type protease, preferably Savinase® or a protein engineered variant of Savinase®, or Alcalase®.

## Patentansprüche

1. Verfahren zur Kristallisation von Enzymen, gekennzeichnet durch die Tatsache, daß eine wäßrige, enzymhaltige Flüssigkeit mit einer Enzymreinheit über 20 % (d.h. das reine Enzym beläuft sich auf mehr als 20 % der Gesamttrockensubstanz in der enzymhaltigen Flüssigkeit) und mit einer Konzentration von reinem Enzymprotein von wenigstens 5 g/l enzymhaltige Flüssigkeit als ein Ausgangsmaterial verwendet wird und daß ein Kristallisationsmittel, welches Na-, K-, Ca- oder Mg- Formiat, -Acetat oder -Nitrat ist, zum Ausgangsmaterial bis zu einer Endkonzentration zugegeben wird, die geringer ist als die Konzentration, die benötigt wird, um die Enzyme in einer amorphen Form auszufällen.

2. Verfahren nach Anspruch 1, wobei das Kristallisationsmittel zum Ausgangsmaterial bis zu einer Endkonzentration zugegeben wird, die einer zugegebenen Menge an Kristallisationsmittel von 0,02 bis 1,7M Kristallisationsmittel entspricht, vorzugsweise von 0,05 bis 1,6M, bevorzugter von 0,10 bis 1,5M.

3. Verfahren nach Anspruch 1 oder 2, gekennzeichnet durch die Tatsache, daß das Enzym eine Protease, Lipase, Amylase, Cellulase, Hemicellulase, Pektinase, Amidase oder Oxidase ist.

4. Verfahren nach Anspruch 3, gekennzeichnet durch die Tatsache, daß das Enzym eine Protease ist und daß die Protease eine Protease vom Subtilisin-Typ ist, vorzugsweise Savinase® oder eine proteintechnologische Variante von Savinase®, oder Alcalase®.

## Revendications

1. Procédé de cristallisation d'enzymes, caractérisé par le fait que l'on utilise comme matière de départ un liquide aqueux contenant une enzyme ayant une pureté en enzyme de plus de 20 % (c'est-à-dire que la quantité d'enzyme pure s'élève à plus de 20 % de la matière sèche totale dans le liquide contenant l'enzyme), et une concentration en protéine enzymatique pure d'au moins 5 g/l du liquide contenant l'enzyme, et en ce que l'on ajoute à la matière de départ un agent de cristallisation, qui est du formate, de l'acétate ou du nitrate de Na, K, Ca ou Mg, à une concentration finale qui est inférieure à la concentration nécessaire pour précipiter les enzymes sous forme amorphe.

2. Procédé selon la revendication 1, dans lequel on ajoute l'agent de cristallisation à la matière de départ à

9

une concentration finale correspondant à une quantité ajoutée d'agent de cristallisation comprise entre 0,02 et 1,7 M d'agent de cristallisation, de préférence entre 0,05 et 1,6 M, de façon plus préférable entre 0,10 et 1,5 M.

3. Procédé selon la revendication 1 ou 2, caractérisé par le fait que l'enzyme est une protéase, une lipase, une amylase, une cellulase, une hémicellulase, une pectinase, une amidase ou une oxydase.

4. Procédé selon la revendication 3, caractérisé par le fait que l'enzyme est une protéase et que la protéase est une protéase du type subtilisine, de préférence la Savinase® ou une variante de la Savinase® obtenue par génie protéique, ou l'Alcalase®.

HCOOH/HAc (20% solution)
KAc
(70% solution)

Crystallization tank
6-7% KAc, 20-25°C
12-20 hours

Mother liquor

Crystal harvest
(filter press)

Flush water
with NaAc

Flush
water

Crystal cake

# Fig. 1

Fig. 2

EP 0 506 866 B1